# NEUE EUROPÄISCHE PATENTSCHRIFT

(11) **EP 1 505 946 B2**
(45) Veröffentlichungstag und Bekanntmachung des Hinweises auf die Entscheidung über den Einspruch: **19.10.2011**
(45) Hinweis auf die Patenterteilung: 25.07.2007
(21) Anmeldenummer: 03749851.6
(22) Anmeldetag: 06.03.2003
(51) Int. Cl.: A61K 8/81, A61K 8/84, A61Q 1/00, A61Q 1/10

(54) **KOSMETISCHE ZUBEREITUNG**
COSMETIC PREPARATION
COMPOSITION COSMETIQUE

(30) Priorität: 10.05.2002 DE 20207329 U
(43) Veröffentlichungstag der Anmeldung: 16.02.2005
(73) Patentinhaber: Schwan-STABILO Cosmetics GmbH & Co., 90562 Heroldsberg (DE)
(72) Erfinder: ZECH, Christina, Kaufering 86916 (DE)
(74) Vertreter: Leissler-Gerstl, Gabriele
(86) Internationale Anmeldenummer: PCT/EP2003/002327
(87) Internationale Veröffentlichungsnummer: WO 2003/094868

(56) Entgegenhaltungen:
- EP-A- 0 745 372
- EP-A- 0 873 748
- EP-B1- 0 992 234
- EP-B1- 1 064 920
- EP-B1- 1 093 802
- WO-A-01/13884
- DE-T2- 69 902 740
- Römpp Chemie Lexikon, 9. Auflage ( 1995 ) Seite 1744 und 2195

## Beschreibung

Die Erfindung betrifft eine Zubereitung zum Auftragen auf die Haut oder Haar, Wimpern und/oder Angenbrauen sowie deren Verwendung, ein Verfahren zu ihrer Herstellung und eine Vorrichtung zu deren Auftrag.

Die erfindungsgemäße Zubereitung kann zu dekorativen Zwecken, zum Schminken der Haut, verwendet werden. Sie kann auch auf Haare, Wimpern und Augenbrauen oder auch deren künstliche Nachbildungen aufgebracht werden, um diesen gewünschte Eigenschaften zu verleihen. Es ist bekannt, Keratinfasern mit einem Überzug zu überziehen, der Fülle und Volumen, eine schöne Form und/oder auch Farbe verleiht. Beispielsweise können Wimpern mit einem farbigen Überzug versehen werden, der sie hervorhebt, in Form bringt und gegebenenfalls auch verlängert. Weiterhin ist es bekannt, Haare mit einem Überzug zu versehen, der die Haare glättet, ihnen Volumen gibt oder gegebenenfalls auch einzelne Strähnen anfärbt.

Zubereitungen zum Schminken der Haut oder der Semi-Schleimhäute sind im allgemeinen bekannt in Form von Stiften, als in kleine Metallpfännchen eingegossene Pasten, als weiche Pasten oder in Form von losen oder gepreßten Pudern. Stifte oder Pasten liegen meist in wasserfreier Form vor als Mischungen aus pflanzlichen, tierischen oder synthetischen Ölen, Fetten und Wachsen, in welchen eine Puderphase aus für Kosmetika zugelassenen Pigmenten, Perlglanzmitteln und Füllstoffen wie Talkum, Kaolin oder amorphem Siliciumdioxid dispergiert ist. Lose oder gepreßte Puder bestehen meist aus Mischungen aus für Kosmetika zugelassenen Pigmenten, Perlglanzmitteln und Füllstoffen, welche mittels eines Bindemittels auf Ölbasis oder in Form einer Emulsion in eine handhabbare oder verarbeitbare Form gebracht werden.

Alle diese Zubereitungen weisen den Nachteil auf, daß sie nur ungenügend am aufgetragenen Ort haften und sich leicht auf andere Materialien wie Geschirr, Besteck, Glas, Textilien oder Haut übertragen lassen. Hieraus ergibt sich die Notwendigkeit, diese Zubereitungen wiederholt aufzutragen. Da Öle auf der Haut und auf Semi-Schleimhäuten spreiten und zudem auch ein unterschiedliches Spreitvermögen aufweisen, können Inhaltsstoffe dieser Zubereitungen, insbesondere Pigmente aus Lippenstiften in die Feinfältelung der Haut um die Lippen einwandern und so störende, weil unschöne, Strukturen erzeugen. Durch das Sebum der Haut und durch Transpiration können diese Effekte provoziert oder verstärkt werden. Puderförmige Lidschatten beispielsweise können durch die Bewegung der Lider und durch Einwirkung von Sebum insbesondere in die Falten des Augenlides wandern und hierdurch streifenförmige Strukturen erzeugen.

Seit etwa 1978 wurde versucht, wachsbasierte Kosmetikstifte, insbesondere solche mit in spitzbare Umhüllungen eingegossenen Minen, auf pigmentierten Wachsmischungen aufzubauen, welche einen deutlichen Anteil an flüchtigen Ölen, vorzugsweise flüchtigen Siliconölen wie Cyclomethiconen oder Kohlenwasserstoffen wie Isoparaffinen oder deren Mischungen, enthielten. Diese Zubereitungen wurden zeitweise noch durch Zusätze nicht flüchtiger Silicone wie Alkyldimethicone oder Phenyltrimethicone in ihren Gebrauchseigenschaften verbessert. Insbesondere bei Verwendung von mit Metalloxiden beschichteten Glimmern konnten sehr geschmeidige und hochglänzende Zubereitungen mit sehr guter Haltbarkeit erzielt werden, die im Markt vom Verbraucher sehr gut angenommen wurden.

Nachteilig war jedoch hierbei der Gehalt an flüchtigen Bestandteilen, was zum einen eine sehr gute Abdichtung der verwendeten Stiftmaterialien, wie Umhüllung, Verschlußkappe und Endkappe verlangte und zum anderen gelegentlich zu Beanstandungen durch die Verbraucher führte, wenn solche Kosmetikstifte längere Zeit ohne Verschlußkappe offen gelagert wurden, weil sie dann wegen der Verdunstung des flüchtigen Anteils schrumpften und sich bis zum Verlust der Gebrauchsfähigkeit verhärteten. Zum anderen empfanden Verbraucherinnen wasserfreie Zubereitungen auf Basis von Ölen, Fetten und Wachsen als unangenehmen Film auf der Haut oder auf Semi-Schleimhäuten, insbesondere dann, wenn diese Zubereitung in einer dickeren Schicht aufgetragen wurde.

Es wurde daher schon versucht, wäßrige oder wäßrig-alkoholische Zubereitungen in Form von polymerhaltigen Dispersionen herzustellen, welche keine Öle, Fette oder Wachse mehr enthielten, die die vorstehend geschilderten Nachteile nicht mehr aufweisen sollten und die auf der Haut oder auf Semi-Schleimhäuten elastische und mehr oder minder wasserfeste Filme bildeten. So beschreibt beispielsweise G.A. Nowak, in "Die kosmetischen Präparate", 1. Auflage 1969 auf Seiten 588 und 589 verschiedene Rezepturen von Eyelinern oder flüssigem Make-up, welche auf synthetischen Filmbildnern wie Polyvinylpyrrolidon (PVP) oder Carboxymethylcellulose oder natürlichen Filmbildnern wie Schellack, Traganth oder Gummi arabicum aufgebaut sind. Auf Seite 441 beschreibt G.A. Nowak Lippenlacke auf Basis von Lösungsmitteln wie Ethylalkohol oder Isopropanol, welche als Filmbildner Ethylcellulose, Celluloseacetat, Schellack, Methylabietat oder PVP enthalten. Solche Zubereitungen haben sich jedoch wegen des Gehaltes an Lösungsmitteln offensichtlich in der Praxis nicht bewährt. EP-A 0 793 957 beschreibt eine Zusammensetzung, die auf die Haut, die Semi-Schleimhäute oder die Schleimhäute aufgetragen werden kann, welche eine wäßrige Dispersion von Partikeln eines filmbildenden Polymers enthält, welches auf einem Träger einen Film mit einer Härte unter 110 (bestimmt nach der Norm NF-T-30-016) bildet. In den Beispielen beschrieben wird jeweils ein Eyeliner und ein Lippenrouge auf Basis von Polyurethandispersionen. Diese Zusammensetzungen sollen einen auf der Unterlage gut haftenden Film bilden, welcher weich und flexibel ist, den Bewegungen der Haut folgt und nicht rissig wird und sich nicht ablöst. Diese Zusammensetzungen sollen auf der Haut nicht wandern und sich nicht auf andere Materialien übertragen.

Weiterhin ist aus EP-A 10 104 18 eine wachsfreie Gelzusammensetzung bekannt, die Polyurethanpolymere als Filmbildner enthält. Diese Zusammensetzung wird als Mascara verwendet und soll länger als einen Tag auf den Wimpern haften. Zur Einstellung der Viskosität wird für diese Zusammensetzungen bevorzugt eine pyrogene Kieselsäure verwendet, die jedoch, insbesondere bei einem stärkeren Auftrag, zu einem Abbröckeln der Masse führt.

WO 01/13884 beschreibt Haarsprays enthaltend Polyurethane und Polyacrylate, jedoch in wasserfreien Zusammensetzungen und wässrige Maskara enthaltend Polyacrylate, jedoch ohne Polyurethane.

Auch EP-A 12 492 25 beschreibt eine wachsfreie Gelzusammensetzung, die wasserfest sein soll und mehr als zwei Tage halten soll. Diese Zusammensetzung soll insbesondere als Färbemittel für Haare, Wimpern und Augenbrauen eingesetzt werden und soll nach dem Auftragen und Trocknen einen glatten Überzug bilden. Nachteilig hier ist allerdings , dass die in der Rezeptur enthaltene pyrogene Kieselsäure die Zusammensetzung schon in geringerer Konzentration stark verdickt und den getrockneten Film stumpf erscheinen lässt. Da sie zudem nicht hydrophobiert ist, fördert sie die Wasseraufnahme des Films und reduziert so die Wasserfestigkeit und damit seine Haltbarkeit.

Weiterhin beschreibt US-A 6 458 390 eine Makeup-Zusammensetzung, die Eisenoxidpigmente und Filmbildner enthält und lang haftend sein soll. Allerdings ist es für diese Zusammensetzung notwendig, dass ein spezielles Siliconacrylat-Copolymer gelöst in einem flüchtigen Lösungsmittel eingesetzt wird. Demgegenüber wird erfindungsgemäß eine wässrige Dispersion eingesetzt. Produkte auf wässriger Basis können die Inhaltsstoffe entweder gelöst oder dispergiert enthalten. Im letzteren Fall muss dafür Sorge getragen werden, dass bei nicht gelösten Inhaltsstoffen, wie z.B. Pigmenten, diese sich nicht mit der Zeit absetzen, und daher zu unbrauchbaren Produkten führen.

Weiterhin besteht bei all diesen Produkten die Anforderung, dass sie, nachdem sie aufgetragen worden sind, an Ort und Stelle bleiben und nicht abfärben, sich lösen, abbröckeln oder hinwegwandern. Die Produkte sollen im Idealfall beständig sein sogar beim Baden oder Duschen, d.h. bei Kontakt mit Wasser, ggf. auch in Verbindung mit Shampoos, Duschpräparaten oder Seife. Speziell für Wimperntusche ist es wünschenswert, dass diese auf den Wimpern bleibt, auch wenn sie mit Wasser in Berührung kommt. Darüber hinaus soll sie beim Darüberreiben nicht abgehen und insbesondere nicht die Umgebung des Auges anfärben. Außerdem soll der Überzug so aufgebaut sein, dass er die Inhaltsstoffe festhält, damit auch nicht Anteile davon auswandern können und die Umgebung anfärben können.

Eine weitere Aufgabe der Erfindung war es, eine kosmetische Zubereitung bereitzustellen, die auf Haar, Wimpern und/oder Angenbrauen aufgetragen werden kann und dort längere Zeit, z.B. mehr als 8 Stunden und insbesondere mehr als einen Tag lang haftet, am aufgetragenen Ort verweilt, ohne durch Wasser, Schweiß oder Berührung mit Gegenständen abgetragen zu werden, andererseits aber auch leicht wieder abnehmbar ist. Weiterhin war es Aufgabe der Erfindung, eine wachsfreie Zusammensetzung bereitzustellen, die einen dauerhaften und glänzenden Überzug liefert. Darüber hinaus sollte eine Zusammensetzung bereitgestellt werden, die leicht auftragbar ist und eine hohe Lagerstabilität aufweist.

Zur Lösung der Aufgabe wird erfindungsgemäß eine Mischung aus wenigstens zwei wäßrigen Dispersionen von filmbildenden Polymeren eingesetzt, die zu einer besseren Haftung und längeren Lagerfähigkeit führt.

Die erfindungsgemäße Zubereitung zum Auftragen auf die Haut oder Haar, Wimpern und/oder Angenbrauen auf wässriger Basis enthält somit eine Mischung aus mindestens zwei wässrigen Dispersionen von flmbildenden Polymeren, von denen eines ein Polymer auf Polyurethanbasis und das andere ein Polymer auf Acrylbasis ist, sowie gegebenenfalls übliche Inhaltsstoffe. In einer bevorzugten Ausführungsform, die insbesondere zum Auftragen auf Haar, Wimpern und/oder Angenbrauen geeignet ist, hat die Zubereitung eine Viskosität, gemessen bei 25°C und mit 200 s⁻¹, im Bereich von 0,02 bis 3,8 Pa·s.

Vorteilhafte Ausführungsformen ergeben sich aus den Unteransprüchen.

Die erfindungsgemäße Zubereitung liegt in Form einer wäßrigen Dispersion vor, welche nach dem Auftragen zu einem wasserfesten Film trocknet und exzellent an der aufgetragenen Stelle haftet. Dieser Film ist elastisch und dehnbarund kann daher den Bewegungen der Haut und Haar, Wimpern und/oder Angenbrauen der Haare folgen. Andererseits zieht er sich beim Trocknen weder zusammen noch schrumpft er erheblich, sodass er kein Spannungsgefühl auf der Haut Haar, Wimpern und/oder Angenbrauen erzeugt und auch nicht abbröckelt. Er wird auch nicht rissig oder körnig und ist so elastisch und haltbar, dass er sich nicht vom Rand des Auftrags her oder gar als Ganzes ablöst - er bleibt vielmehr "wie eine zweite Haut" an der Auftragsstelle. Seine Bestandteile wandern nicht, auch nicht unter dem Einfluß von Sebum oder Transpiration, und seine Bestandteile lassen sich auch nicht auf andere Materialien übertragen. In der Regel zeigt der Film schon beim Trocknen, spätestens aber nach komplettem Trocknen, einen hohen Oberflächenglanz und hohes Reflexionsvermögen. Der Film ist nach dem kompletten Trocknen so stabil, daß er durch einen eventuellen zweiten Auftrag der Zubereitung nicht nachteilig verändert wird. Keinesfalls darf der getrocknete Film kleben oder ein klebriges Gefühl ergeben.

Falls die erfindungsgemäße Zubereitung Färbemittel enthält, müssen diese solche Korngrößen, Oberflächenbeschaffenheiten und Formen aufweisen, dass die Färbemittel die Filmbildung nicht nachteilig beeinflussen, obwohl sie - gewissermaßen als "Fremdkörper" - die Ausbildung des Films stören. Als für kosmetische Zubereitungen geeignete Färbemittel können anorganische Pigmente, z.B. gelbe, rote oder schwarze Eisenoxide, Ultramarine, Chromoxidgrün, Chromoxidhydratgrün, Ruß (Carbon Black) und/oder ggf. organische Pigmente, Verlackungen organischer Farbstoffe, plättchenförmige Metallpulver, z.B. passiviertes Aluminium, Messing, Bronze, Kupfer, Silber oder Gold, Glimmer, mit Metalloxiden, z.B. mit Titandioxid, Eisenoxiden, Chromoxid, Chromoxidhydrat beschichtete Glimmer, plättchenförmige Zubereitungen auf Basis von Siliciumdioxid, Aluminiumoxid oder Glas, welche ggf. auch mit Metalloxiden, z.B. Titandioxid, Eisenoxiden, Titandioxid, Eisenoxiden, Chromoxid, Chromoxidhydrat beschichtet sein können und deren Mischungen genannt werden. Die genannten synthetischen plättchenförmigen Materialien haben dabei den Vorteil, daß sie in gleichmäßiger Schichtdicke herstellbar sind und sich schon bei den Vorlieferanten einfacher weiterverarbeiten lassen.

Erfindungsgemäß wird eine Zusammensetzung bereitgestellt, die sich sehr leicht auftragen lässt und, sobald sie aufgetragen ist, lange an Ort und Stelle haftet, ohne sich in negativer Weise zu verändern, auf andere Gegenstände überzugehen, in benachbarte Bereiche zu wandern oder abzufärben oder abzubröckeln. Außerdem ist die Zusammensetzung trotz ihrer geringen Viskosität sehr lange lagerfähig, ohne dass sich Inhaltsstoffe absetzen. Der mit der erfindungsgemäßen Zusammensetzung erhaltene Überzug oder Film weist den für ein Kosmetikum wünschenswerten Glanz auf.

All diese wünschenswerten und vorteilhaften Eigenschaften werden erzielt, wenn in einer wässrigen Zusammensetzung mindestens eine Dispersion eines Polyurethanpolymers in Kombination mit mindestens einer Dispersion eines Acrylpolymers verwendet wird. Dazu werden die Dispersionen in solchen Anteilen eingesetzt, dass eine Zubereitung mit einer Viskosität im gewünschten Bereich erhalten werden kann. Überraschenderweise wurde festgestellt, dass eine Kombination dieser beiden Inhaltsstoffe dazu führt, dass sich ein stabiles Produkt mit geringer Viskosität bilden lässt, dessen Haftung an Haut und Haar, Wimpern und/oder Angenbrauen sehr vorteilhaft ist. Nur wenn jeweils von beiden genannten Polymerklassen mindestens ein Vertreter enthalten ist, werden diese vorteilhaften Eigenschaften erzielt. Darüberhinaus werden diese vorteilhaften Eigenschaften erzielt, ohne dass ein Wachs enthalten sein muss. Ein Wachs kann dieser Zubereitung zwar zugesetzt werden, ist aber zur Erzielung der gewünschten Eigenschaften nicht notwendig und daher nicht bevorzugt.

Wesentlich für die erfindungsgemäße Zubereitung ist somit der Gehalt einer wässrigen Dispersion mindestens eines Polykondensates ausgewählt unter den anionaktiven, kationaktiven, nichtionogenen oder amphoteren Polyurethanpolymeren bzw. -copolymeren. Das Polyurethan kann dabei vorteilhafterweise ausgewählt werden aus der Gruppe der Polyester-Polyurethane, Polyether-Polyurethane, Polyurethan-Polyvinylpyrrolidone, Acrylpolyurethane und siliconhaltigen Polyurethane sowie deren Mischungen.

Das Polyurethan liegt in wässriger Phase in Form von Teilchen vor, wobei deren Größe bevorzugt in einem Bereich von 10 bis 1000 nm, besonders bevorzugt 15 bis 300 nm und insbesondere 30 bis 100 nm liegt.

Geeignet sind alle Polyurethandispersionen, die nach dem Auftragen einen Film bilden. Derartige Polyurethanpolymere sind dem Fachmann bekannt. Als Beispiele können Polyester-Polyurethane, wie sie z.B. von der Firma Noveon (ehemals Fa. BF Goodrich) unter der Bezeichnung Avalure angeboten werden, z.B. Avalure UR-425, Avalure UR-430, Avalure UR-405 und Avalure UR-410, oder auch Produkte, wie sie von der Firma Zeneca unter der Bezeichnung Neorez angeboten werden, genannt werden. Bevorzugt ist das Polyurethan-Polymer ein mit der CTFA-Bezeichnung Polyurethan-2 bezeichnetes.

Das Polyurethanpolymer kommt als wässrige Dispersion zum Einsatz. Bevorzugt wird eine Dispersion des Polymers mit einem Anteil an Polymer von 10 bis 80%, bevorzugt 30 bis 60% in Wasser eingesetzt. Geeignet sind auch Mischungen der oben aufgeführten Polyurethanpolymere, insbesondere Mischungen von Polyester-Polyurethanen und Polyether-Polyurethanen oder auch Mischungen von Polyurethanpolymeren mit Polyharnstoffen, Siliconylacrylaten, PEG/PPG-25/25 und/oder Dimethicone/Acrylates Copolymer.

Der Anteil der wässrigen Polyurethan-Dispersion an der Gesamtzusammensetzung sollte in einem Bereich von 1 bis 60%, bevorzugt 15 bis 50% liegen. Alle Prozentangaben in dieser Beschreibung, den Beispielen und den Ansprüchen beziehen sich immer auf Gewicht, wenn nichts anderes angemerkt wird. Die Polyurethankomponente sorgt bei dem gebildeten Film unter anderem für die Wasserbeständigkeit. Unter einem Anteil von 1 % tritt die gewünschte Wirkung des Polymers nicht mehr ein. Bei mehr als 60% ist die Einstellung der Viskosität und die Einarbeitung von Färbemitteln erschwert.

Die zweite wesentliche Komponente der erfindungsgemäßen Zusammensetzung ist mindestens ein Polymer auf Acrylbasis, das für die Haftung der Zusammensetzung, für Glanz und Abriebfestigkeit sorgt. In der Regel handelt es sich um ein durch radikalische Polymerisation erhaltenes Polymer, das vorzugsweise ein Acrylates Copolymer oder Acryl- und/oder Vinylpolymer und/oder Acryl- und/oder Vinyl-Copolymer ist. Diese Polymere werden insbesondere durch die radikalische Polymerisation von geeigneten Monomeren, bevorzugt im Wege der Emulsionspolymerisation, gewonnen. Grundsätzlich ist es auch möglich, die durch radikalische Polymerisation gewonnenen Polymere in einem geeigneten Lösungsmittel aufzulösen und die Lösung weiterzuverwenden. Als geeignete Monomere seien Acrylsäure, Crotonsäure, Maleinsäureanhydrid oder Methacrylsäure beispielhaft genannt. Acrylpolymere und -Copolymere können aber auch durch Polymerisation oder Copolymerisation von Monomeren erhalten werden, die z.B. ausgewählt sind unter Monomeren wie Methylacrylat, Ethylacrylat, Butylacrylat, Methylmethacrylat, Ethylmethacrylat, Butylmethacrylat, Butylacrylamid, Ethylhexylacrylamid und dergleichen.

Auch Acrylpolymere werden als wässrige Dispersionen eingesetzt, deren Teilchengröße im selben Bereich liegt, wie bei dem Polyurethanpolymer. Als Acrylpolymer oder Acrylates Copolymer können sowohl Arylacrylat-Copolymere als auch Alkylacrylat-Copolymere eingesetzt werden, wobei im letzteren Fall der Alkylanteil 1 bis 30 C-Atome aufweisen kann. Das Acrylates Copolymer ist ein Polymer, das bevorzugt durch Polymerisation mindestens eines Monomers, das eine ethylenisch ungesättigte Bindung enthält, und ausgewählt ist aus α,β-ethylenisch ungesättigten Carbonsäuren, α,β-ethylenisch ungesättigten Carbonsäureestern und α,β-ethylenisch ungesättigten Carbonsäureamiden und Kombinationen von zwei oder mehr dieser Monomere erhalten wird. Bevorzugt kommen als α,β-ethylenisch ungesättigten Carbonsäuren Acrylsäure, Methacrylsäure und deren Ester und Amide sowie, Crotonsäure, Maleinsäure und Itaconsäure zum Einsatz. (Meth)acrylsäure und Crotonsäure werden bevorzugt verwendet. Besonders bevorzugt sind Acrylsäure und Methacrylsäure, sowie deren Ester.

Als α,β-ethylenisch ungesättigten Carbonsäureester kommen insbesondere (Meth)-acrylsäureester in Betracht, deren Esteranteil ein C₁-C₃₀-Alkylrest oder C₆-C₁₀-Arylrest ist. Die Alkyl- bzw. Arylanteile können dabei zusätzlich substituiert sein, z.B. mit Hydroxyresten. Bevorzugte Acrylate, die verwendet werden können, schließen Methylmethacrylat, Ethylmethacrylat, Butylmethacrylat, Isobutylmethacrylat, 2-Ethylhexylmethacrylat, Laurylmethacrylat und Cyclohexylmethacrylat ein. Bevorzugte Arylmethacrylate, die verwendet werden können, schließen Benzylacrylat und Phenylacrylat ein. Besonders geeignet sind Copolymere aus Styrol und (Meth)acrylsäure(estern).

Weiterhin können auch Carbonsäureamide verwendet werden. Bevorzugt können N-Alkyl- oder N-Aryl(meth)acrylamide genannt werden, deren Alkylanteil 2 bis 15 C-Atome aufweisen kann bzw. deren Arylanteil 6 bis 10 C-Atome aufweisen kann. Als bevorzugt können hier N-Ethylacrylamid, N-t-Butylacrylamid, N-t-Octylacrylamid und N-Undecylacrylamid genannt werden.

Weiterhin können auch Copolymere der genannten Monomere mit anderen olefinisch ungesättigten Monomeren verwendet werden. Beispiele hierfür sind Acrylcopolymere, welche durch Copolymerisation von Acrylsäure oder Methacrylsäure mit Monomeren wie Butadien, Styrol oder Vinylestern wie Vinylacetat oder Vinylbenzoat erhalten wurden. Bevorzugt werden Copolymere verwendet, die durch Copolymerisation von Acrylsäure oder Methacrylsäure mit Styrol- oder Vinylverbindungen erhalten werden.

Das Acrylates Copolymer wird in Form einer wässrigen Dispersion eingesetzt, wobei die wässrige Dispersion das Copolymer in einem Anteil von 20 bis 70%, besonders bevorzugt 25 bis 40% enthält. Der Anteil des Acrylates Copolymers bzw. der wässrigen Dispersion des Acrylates Copolymers an der Gesamtzusammensetzung beträgt 1 bis 60%, jeweils bezogen auf das Gewicht der Gesamtzusammensetzung und bevorzugt 15 bis 35%. Unter einem Anteil von 1 % wird die gewünschte Abriebfestigkeit nicht erreicht. Bei einem Anteil von mehr als 60% wird das Quellvermögen des getrockneten Films erhöht und damit seine Wasserfestigkeit verringert, was zu schlechten Gebrauchseigenschaften führt. Zudem ist die Einarbeitbarkeit von Färbemitteln stark erschwert.

In einer bevorzugten Ausführungsform der Erfindung wird als Acryl-Komponente eine Mischung aus mindestens zwei verschiedenen Polymeren auf Acrylbasis eingesetzt, von denen eines bevorzugt ein Acrylates Copolymer ist und das andere ein Copolymer von Acrylsäure/Methacrylsäure-Monomeren und ethylenisch ungesättigten Arylmonomeren ist. Bevorzugt ist das letztere Acrylpolymer ein styrolhaltiges Acrylates Copolymer. Besonders bevorzugt wird hierbei Styrene Acrylates Ammonium Methacrylate Copolymer eingesetzt.

Auch diese Komponente wird bevorzugt in Form einer wässrigen Dispersion verwendet, wobei die Dispersion in der Regel 30 bis 50% Polymer in Wasser enthält. Der Anteil dieses speziellen Aryl/Acrylat-Copolymers an der Gesamtzusammensetzung beträgt 1 bis 60%, wobei ein Anteil im Bereich von 15 bis 35% und insbesondere 20 bis 30% besonders bevorzugt ist.

In einer zum Auftragen auf Hautanhangsgebilde besonders geeigneten Ausführungsform bilden die wässrigen Polymerdispersionen 50 bis 90 %, bevorzugt 55 bis 75 % der Gesamtzusammensetzung. Dabei werden bevorzugt eine Polyurethandispersion, eine Acrylates Copolymer-Dispersion und eine Styrol/Acryl-Dispersion eingesetzt. Besonders bevorzugt wird diese Kombination in solchen Anteilen verwendet, dass der Polymeranteil zu etwa 35 bis 55 % aus Polyurethan, zu etwa 15 bis 25 % aus Acrylates Copolymer-Dispersion und dem Rest aus Styrol/Acryl-Dispersion gebildet wird.

Um besonders gute Eigenschaften zu erzielen werden die Polymere bevorzugt so ausgewählt, dass 30 bis 60 % der Polymerdispersionen von im getrockneten Zustand wasserfesten Polymeren oder Copolymeren eine Glasübergangstemperatur unter 10°C haben. Besonders bevorzugt zeigt bei 5 bis 30 % einer oder mehrerer der Dispersionen von statistischen oder Blockcopolymeren ein Block oder Abschnitt im getrockneten Zustand eine Glasübergangstemperatur von mehr als 30°C.

Außerdem wurde festgestellt, dass besonders vorteilhafte Ergebnisse erzielt werden, wenn alle filmbildenden Polymeren eine Mindestfilmbildungstemperatur unterhalb 30°C und bevorzugt unterhalb der Raumtemperatur haben.

Die erfindungsgemäße Zubereitung kann zusätzlich zu den oben genannten Polymerkomponenten noch weitere Polymere enthalten, solange diese kompatibel sind und die Eigenschaften der erfindungsgemäßen Komponenten nicht beeinträchtigen. So können noch sog. Hybridpolymere, welche durch Copolymerisation als Acryl- oder Vinylpolymere mit Siliconsegmenten oder Silicongruppen erhältlich sind, enthalten sein. Bekannt und im Handel erhältlich sind auch Polykondensate mit Siliconsegmenten oder Silicongruppen.

Geeignet sind auch Polyester, Polyesteramide oder Polyamide. Polyester können nach den dem einschlägig befaßten Fachmann bekannten Methoden durch Polykondensation von aliphatischen oder aromatischen Dicarbonsäuren mit aliphatischen oder aromatischen Diolen oder mehrwertigen Alkoholen erhalten werden.

Beispielhaft genannt seien hier Polykondensate aus Adipinsäure, Bernsteinsäure, Glutarsäure, Sebacinsäure, Terephthalsäure oder Isoterephthalsäure mit Diolen wir Ethylenglykol, Diethylenglykol, Propylenglykol oder mehrwertigen Alkoholen wir Glycerin, Trimethylolpropan, Pentaerythrit, Mannitol, Sorbitol oder Xylitol oder polymeren Weichsegmenten wie Poly-THF. Polyesteramide gewinnt man entsprechend durch die Polykondensation von Dicarbonsäuren mit Diaminen oder Aminoalkohlen.

Als geeignete natürliche Polymere seien nur beispielhaft genannt: Schellack, Dammarharz, Kopal, Elemi, Traganth, Gummi arabicum, Xanthan oder Cellulosederivate, welche ggf. chemisch modifiziert sein können, und deren Mischungen.

Ein weiteres wesentliches Merkmal der erfindungsgemäßen Zusammensetzung ist die Viskosität, die in einem Bereich von 0,02 bis 3,8 Pa·s, gemessen bei 25°C und 200 s⁻¹ liegt. Die Viskosität kann in an sich bekannter Weise unter Verwendung eines Rheometers gemessen werden. Die Messung wird geeigneterweise mit einem Rheometer, Typ BOHLIN CVOR (Messsystem Platte/Platte) bei 25°C und bei einer Scherrate von 200 s-1 ohne Vorscherung gemessen. Verwiesen sei hier zusätzlich auf die Norm DIN 53018, Teil 1 und Teil 2.

Überraschenderweise wurde festgestellt, dass die erfindungsgemäße Zusammensetzung trotz der geringen Viskosität sehr gut haltbar und lange lagerfähig ist und aufgrund ihrer Struktur auch teilchenförmige Inhaltsstoffe in Dispersion halten kann, so dass keine Entmischung oder ein Absetzen der Färbemittel eintritt.

Die erfindungsgemäße Zusammensetzung kann außer den oben beschriebenen filmbildenden Polymeren je nach den gewünschten Eigenschaften und nach Einsatzgebiet weitere übliche Inhaltsstoffe enthalten.

Wenn die erfindungsgemäße Zusammensetzung zum Anfärben vorgesehen ist, zur Färbung von Haaren, einschließlich Wimpern und/oder Augenbrauen, oder aber als dekorative Augenkosmetik, z.B. als Eyeliner, enthält sie weiterhin Färbemittel. Färbemittel für derartige Zusammensetzungen sind an sich bekannt und die für Kosmetika geeigneten können auch für die erfindungsgemäße Masse eingesetzt werden.

Zur Einfärbung der erfindungsgemäßen Zubereitung können anorganische Pigmente wie Titandioxid, Zinkoxid, Eisenoxide, Chromoxidgrün, Chromoxidhydratgrün, Ultramarine, Ruß (Carbon Black), organische Pigmente wie Carmin und seine Salze oder Phthalocyanin, Glimmer, mit Metalloxiden wie Titandioxid, Eisenoxid, Chromoxid beschichtete Glimmer, Bismuthoxychlorid oder mit Metalloxiden beschichtetes Bismuthoxychlorid, plättchenförmige Zubereitungen auf Basis von Siliciumdioxid, Aluminiumoxid oder Glas, welche ggf. auch mit Metalloxiden, z.B. Titandioxid, Eisenoxiden, Titandioxid, Eisenoxiden, Chromoxid, Chromoxidhydrat beschichtet sein können, plättchenförmige Metallpulver wie passiviertes Aluminium, Bronze, Messing, Kupfer, Silber, Gold, Barium-, Aluminium-, Strontium-, Calcium- oder Zirkoniumverlackungen organischer Farbstoffe oder deren Mischungen verwendet werden. Bei der Auswahl des jeweils geeigneten Färbemittels sollte auch die für die Herstellung von Kosmetika nationale oder regionale Gesetzgebung beachtet werden, da in manchen Ländern Färbemittel, die mit den Schleimhäuten des Auges in Berührung kommen, zugelassen sein müssen. In Deutschland beispielsweise, ist die Zulässigkeit von Färbemittel durch den Anhang 3 zu § 3 der Kosmetik-Verordnung geregelt.

Zum Anfärben von Wimpern, Augenbrauen und Haaren, können sowohl organische lösliche Farbstoffe als auch Pigmente eingesetzt werden. Pigmente sind für die erfindungsgemäße Zusammensetzung bevorzugt. Es können sowohl normale Pigmente als auch beschichtete Pigmente eingesetzt werden. Die Pigmente haben bevorzugt eine kleine Teilchengröße, bevorzugt in einem Bereich von 20 nm bis 150 µm. Hierzu werden die Pigmente in üblicher Weise fein vermahlen oder ggf. micronisiert eingesetzt.

Falls die erfindungsgemäße Zusammensetzung zur Anfärbung von Augenwimpern und Augenbrauen verwendet werden soll, sind als Pigmente z.B. schwarzes Eisenoxid oder Ultramarinblau geeignete Färbemittel. Der Anteil des Pigmentes richtet sich nach der gewünschten Färbung und geeignete Mengen sind dem Fachmann an sich bekannt. Für die erfindungsgemäße Zusammensetzung kommt z.B. ein Anteil von 0 bis 30%, insbesondere 5 bis 25% in Betracht. Für spezielle Farbstoffe, mit denen besondere Effekte erzielt werden sollen, sind jedoch auch Anteile außerhalb dieser Bereiche gegebenenfalls möglich.

Die Lagerfähigkeit der erfindungsgemäßen Produkte kann verbessert werden, indem vorzugsweise Pigmente mit hydrophober, physikalisch oder chemisch haftender Beschichtung verwendet werden. Beschichtungen für Pigmente sind an sich bekannt und bedürfen hier keiner näheren Erläuterung. Geeignet sind beispielsweise siliconhaltige Beschichtungen mit Dimethicone oder anderen Siliconen oder Beschichtungen mit Titantriisostearat. Der Einsatz von beschichteten Pigmenten hat den weiteren Vorteil, dass ein Benetzen oder Quellen des gebildeten Films bei Kontakt mit Wasser minimiert wird. Dadurch wird die Dauerhaftigkeit und Beständigkeit des gebildeten Films weiter verstärkt. Selbst wenn der Film bei Kontakt mit Wasser etwas quillt, bleibt er, wenn er in dem gequollenen Zustand nicht mechanischer Reibung ausgesetzt wird, völlig intakt und stabil.

Bei Verwendung von stark hydrophob beschichteten Pigmenten ist es empfehlenswert, der Zusammensetzung auch eine oberflächenaktive Substanz zuzugeben, gegebenenfalls in Kombination mit einem polymeren Netzmittel. Derartige oberflächenaktive Substanzen und Netzmittel tragen zur Wasserkompatibilität der Teilchen bei.

Als Beispiele für geeignete oberflächenaktive Mittel können niedermolekulare Tenside, wie Sarcosinate, nichtionische und oder anionaktive Tenside, wie PEGmodifizierte Fettsäuren und Fettalkohole, Natriumlaurethsulfat, Solubilisatoren, wie sie auch zur Kompatibilisierung von UV-Substanzen üblich sind, und/oder Esteröle genannt werden.

Das niedermolekulare Tensid wird, falls vorhanden, bevorzugt in einem Anteil von 0,1 bis 3%, jeweils bezogen auf das Gewicht der Gesamtzusammensetzung, verwendet.

Als polymere Netzmittel geeignet sind z.B. Silicontenside und amphiphile Siliconylacrylate, sowie PVP-Derivate, z.B. VP/Eicosen- und VP/Hexadecen-Copolymer, Fluortenside, Cholesterolester, Lecithin, und Acrylat-Copolymere, z.B. ein Acrylat-Blockcopolymer und andere bekannte Polymertenside. Besonders bevorzugt wird als polymeres Netzmittel ein acrylhaltiges Netzmittel eingesetzt, um die Kompatibilität zu verbessern. Das polymere Netzmittel wird, falls vorhanden, geeigneterweise in einem Anteil von 0,05 bis 5%, bevorzugt 0,1 bis 3 %, verwendet. In einer bevorzugten Ausführungsform wird ein nichtionogenes Netzmittel, insbesondere ein Dimethicone Copolyol oder ein nichtionogenes polymeres Netzmittel, wie z.B. ein Acrylat- oder Polyethylen-Blockcopolymer in geringer Menge, vorzugsweise 0,05 bis 3 Gew.-%, ganz besonders bevorzugt 0,3 bis 1,5 Gew.-% eingesetzt. Hierdurch wird bewirkt, daß die Färbemittelteilchen einerseits gut benetzt und andererseits durch molekulare Wechselwirkungen in die Polymerstruktur eingebunden werden. Gleichzeitig wird dadurch bewirkt, daß der Film beim Trocknen besonders glatt verläuft und dadurch einen hohen Oberflächenglanz ausbildet. Dieser kann auch durch den Einsatz von Polymersegmenten mit bekannt gutem Reflexionsvermögen und einer höheren Glasübergangstemperatur T_{g}, wie z.B. Polystyrol, erreicht werden.

In einer bevorzugten Ausführungsform der erfindungsgemäßen Zubereitung, die pigmentiert ist, wird bevorzugt zusammen mit den Pigmenten auch Verdicker eingesetzt, der für eine noch bessere Lagerstabilität sorgt. Da gleichzeitig jedoch die erhaltene Masse nicht bröselig werden soll, sondern glatt aufzutragen sein muss, sind nicht alle bekannten Verdicker hierfür geeignet. Insbesondere pyrogene Kieselsäure ist ungeeignet, da sie nach dem Auftragen der Zusammensetzung zu einem Abbröseln führt. Außerdem soll die erfindungsgemäße Zubereitung eine so geringe Viskosität aufweisen, dass sie frei fließend ist und daher auch in Gefäßen verschiedenster Geometrie aufbewahrt werden kann, ohne dass ein Problem bei der Entnahme besteht. Es wurde überraschenderweise festgestellt, dass eine niedrigviskose Zubereitung erhalten werden kann, wenn eine Verdickermischung eingesetzt wird, die mindestens ein anorganisches Schichtsilicat kombiniert mit einem Xanthangummi enthält. Geeignete Mischungen sind solche aus natürlichen und synthetischen sowie modifizierten Polysacchariden, mineralischen und modifizierten mineralischen Verdickern, Silicatverdickern, Cellulosen und modifizierten Cellulosen, nichtionischen Assoziativverdickern, Copolymerverdickern, wie Hydroxyethylacrylate/Sodium Acryloyl Dimethyltaurate Copolymer, Sodium Acrylate Acryloyl Dimethyltaurate Copolymer, Sodium Acrylate/Acryloyl Dimethyltaurate Copolymer, VP/Dimethiconyl Acrylate/Polycarbamoyl/Polyglycolester. Besonders gute Eigenschaften werden erhalten, wenn eine Mischung aus einem größeren Anteil eines Hydrokolloids oder pflanzlichen Gummis, z.B. Xanthangummi, einem kleineren Anteil eines silicatischen Verdickers und einem geringen Anteil eines Polysaccharids, insbesondere Cellulose, die ggf. auch chemisch modifiziert sein kann, eingesetzt wird. Beispielhaft ist eine Mischung aus 40 bis 70% Xanthangummi, 10 bis 40% mineralischem Verdicker und Rest Cellulose oder Cellulosederivat. Besonders bevorzugt wird als Verdicker eine Mischung aus Cellulose, Silicat und Xanthamgummi in einem Verhältnis von im Bereich von 1:1:4 bis 1:2:6 eingesetzt.

Die Verdickermischung kann je nach gewünschter Viskosität in einem Anteil bis zu 20% zugefügt werden. In der Regel haben sich Anteile im Bereich von 0,2 bis 5%, jeweils bezogen auf das Gewicht der Gesamtzusammensetzung, als geeignet erwiesen. Wenn eine niedrigere Viskosität erwünscht ist, wird natürlich der Verdickeranteil im unteren Bereich eingesetzt, während dann, wenn die Viskosität erhöht werden soll, der Anteil des Verdickers erhöht wird.

Die erfindungsgemäß verwendete Verdickermischung stabilisiert die Pigmente und verhindert damit im Wesentlichen, dass sich die Pigmente absetzen. Damit bleiben die Pigmente auch bei längerer Lagerung homogen verteilt und müssen nicht vor jeder Anwendung durch Aufschütteln redispergiert werden.

Andererseits wird durch die niedrige Viskosität, die trotz Einsatz der Verdickermischung erzielt werden kann, erreicht, dass die Masse auf der Wimper nicht aufträgt und trotzdem gut färbt. Nachteil bei einem dickeren Auftrag ist es, dass sich dabei beim Trocknen Spannungen ausbilden, die dazu führen, dass die Masse nach einiger Zeit abplatzt. Mit der erfindungsgemäßen Zusammensetzung wird dagegen ein Überzug erreicht, der aufgrund seiner Elastizität und seiner gleichmäßigen Schichtdicke lange und dauerhaft am aufgetragenen Ort bleibt.

In einer Ausführungsform kann die erfindungsgemä0ße Zubereitung auch ein oder mehrere übliche und an sich bekannte Sonnenschutzmittel bzw. UV-absorbierende Mittel enthalten, um Haut oder Haar vor Sonneneinstrahlung zu schützen. Häufig wird diese Ausführungsform ohne Pigmentzusatz verwendet. Die Sonnenschutzmittel werden in den für Kosmetika üblichen Mengen eingesetzt.

Falls für die erfindungsgemäße Zusammensetzung eine Konservierung erwünscht ist, können hierfür alle gängigen Konservierungsmittel eingesetzt werden, unter anderem auch Carbamate, z.B. lodpropinylbutylcarbamat. Der Einsatz dieser Konservierungsmittel erfolgt bis zu den gesetzlich zugelassenen Mengen, wie sie beispielsweise in Deutschland durch die Anlage 6 zu § 3a der Kosmetik-Verordnung in der jeweils gültigen Fassung vorgegeben sind.

Um die rheologischen Eigenschaften der erfindungsgemäßen Zusammensetzung und das Auftragen der Zusammensetzung weiter zu verbessern, können gegebenenfalls auch Co-Lösungsmittel, d.h. mit Wasser mischbare Lösungsmittel eingesetzt werden. Geeignet sind hier z.B. ein- und/oder mehrwertige Alkohole mit 2 bis 20 Kohlenstoffatomen, Ester und Ether davon, flüssige, mit Wasser mischbare oder in Wasser leicht dispergierbare Verbindungen wie z.B. N-Methyl-Pyrrolidinon, Dimethylacetamid, Glycerin, Diglycerin, Triglycerin, Butylenglycol, Hexantriol, Propylenglykol, Dipropylenglykol, Hexylenglykole, Gemische aus Dimethicone Copolymer und Caprylic/Capric Triglyceride oder sonstige geeignete Silicontenside, sowie Lösungen von in Wasser leicht löslichen festen Stoffen, die als Feuchthaltemittel aber nicht als Co-Lösungsmittel dienen, wie z.B. Harnstoff, Xylitol, Inositol, Maltitol, Sorbitol, Mannitol, Glucose, Fructose, Sucrose, Lactose, Trehalose, Diglycet-7-malat, Glyceryldimaltodextrin, Sorbitylsilandiol, sowie Aminosäuren oder stickstoffhaltige Verbindungen wie Acetylarginin, , Amidinoprolin, Betain, Chitosanlauroylglycinat, Diglycolguanidinsuccinat, Derivate von Guanidinacetat und Mischungen darau. Bevorzugt werden als Co-Lösungsmittel mehrwertige Alkohole eingesetzt, die gleichzeitig auch als Feuchthaltemittel dienen. Das Co-Lösungsmittel ist üblicherweise in einem Anteil von 0 bis 5% enthalten.

Die erfindungsgemäß verwendeten filmbildenden Polymere haben eine Glasübergangstemperatur in einem Bereich, der einen weichen und elastischen Film auch ohne Weichmacher entstehen lässt. Falls jedoch die Weichheit und Elastizität noch weiter erhöht werden soll, oder auch, wenn das Verlaufen der Masse weiter gefördert werden soll, kann auch ein für kosmetische Massen bekannter Weichmacher zugesetzt werden. Geeignet sind hier sowohl niedermolekulare als auch höher molekulare Weichmacher, die gegebenenfalls in einem Co-Lösungsmittel gelöst oder solubilisiert verwendet werden. Als geeignet kommen Silicontenside, aber auch kurzkettige Esteröle in Betracht. Die Härte des aus der erfindungsgemäßen Zubereitung gebildeten Films kann darüber hinaus auch durch Zusatz von Benzoylbenzoat, Tributylcitrat, Trihydroxypropylcitrat oder Laureth-2-benzoat beeinflußt werden.

Falls vorhanden, werden die Weichmacher in an sich bekannter Weise verwendet, z.B. in einem Anteil von 0 bis 5%.

Um ein Schäumen der Zusammensetzung während der Herstellung oder bei der Anwendung - z.B. im Fall einer Wimperntusche durch den "Luftpumpeneffekt" beim Herausziehen und Wiedereinbringen des Applikators in den Behälter - zu verhindern, was beim Auftragen nachteilig sein kann, können an sich bekannte Entschäumer in geringer Menge zugesetzt werden. Beispiele hierfür sind Siliconverbindungen, wie sie auch für andere kosmetische Zusammensetzungen verwendet werden. Üblicherweise wird eine derartige Siliconverbindung als Emulsion in einem Anteil von ca. 0 bis 5%, bevorzugt 0,1 bis 1 % eingesetzt.

Wenn die aufgetragene Masse wieder entfernt werden soll, so kann dies in einfacher Weise erreicht werden, indem Wasser mit höherer Temperatur, d.h. mit einer Temperatur über 25°C, bevorzugt zwischen 30 und 38°C auf den aufgetragenen Film aufgebracht wird und der Film anschließend durch mechanische Reibung, z.B. mit einem Wattepad, abgenommen wird.

Die erfindungsgemäße Zubereitung kann in an sich bekannter Weise hergestellt werden. Um eine besonders glatte, homogene und gut auftragbare Masse zu erhalten, erfolgt die Herstellung bevorzugt, indem zuerst die Polymerdispersionen vorbereitet und auf eine erhöhte Temperatur erwärmt werden, bevorzugt im Bereich zwischen 45 und 60°C.

Falls eine Verdickermischung eingesetzt wird, wird diese bevorzugt in einem Cosolvens, das beliebig mit Wasser mischbar, aber von Wasser verschieden ist, z.B. einem mehrwertigen Alkohol, Glycerin oder Esteröl, vordispergiert. Beide Mischungen werden dann zusammengemischt und die weiteren Bestandteile in üblicher Weise zugefügt. In die homogene Dispersion können dann die Färbemittel eingearbeitet und gleichmäßig darin verteilt werden.

Diese Masse kann nun in die Vorrichtung, aus der die Zusammensetzung abgegeben werden soll, ohne weiteres abgefüllt werden. Die erfindungsgemäße Zusammensetzung ist so stabil, dass sie sich auch ohne Rühren nicht entmischt oder absetzt. Ggf. kann die Zusammensetzung auch unmittelbar nach der Herstellung noch heiß in die dafür vorgesehenen Behälter abgefüllt werden.

In seltenen Fällen kann sich während des Abkühlvorganges etwas Wasser an der Oberfläche absetzen, das jedoch durch einfaches Einrühren homogen wieder verteilt werden kann. Da die Viskosität der erfindungsgemäßen Zusammensetzung gering ist, kann sie auch im Kalten abgefüllt werden, was vorteilhaft für den Erhalt der Massestruktur ist.

Falls erwünscht, können nach Fertigstellung der Zusammensetzung noch Hilfsmittel, wie Weichmacher, Glanzgeber, Verlaufshilfsmittel etc. zugegeben werden.

Die erfindungsgemäße Zusammensetzung kann zur Behandlung von Haut und Haar, Wimpern und/oder Angenbrauen verwendet werden. Insbesondere ist die erfindungsgemäße Zusammensetzung einerseits zum Schminken der Augen und andererseits zur Behandlung von Wimpern, Augenbrauen und Haaren geeignet.

Ein weiterer Gegenstand der Erfindung ist daher die Verwendung der erfindungsgemäßen Zubereitung zum Schminken der Wimpern und Augenbrauen.

Ein weiterer Gegenstand der Erfindung ist die Verwendung der erfindungsgemäßen Zubereitung zur Behandlung von Haar, Wimpern und Augenbrauen.

Bevorzugt wird die erfindungsgemäße Zubereitung verwendet, um keratinisches Material mit einem Überzug, insbesondere einem färbenden Überzug zu versehen. Dieser Überzug ist lange haltbar und bleibt auch bei Kontakt mit Wasser bestehen. Die Färbung bleibt bis zu 4 Tagen bestehen. Falls erwünscht, kann der Überzug jederzeit wieder abgenommen werden, indem Wasser, bevorzugt höherer Temperatur, aufgebracht wird und nach wenigen Minuten durch mechanisches Reiben der Überzug abgenommen wird.

Falls die erfindungsgemäße Zubereitung zum Auftrag auf keratinisches Material verwendet wird, so kann der Auftrag bevorzugt mit einem Auftragssystem aufgetragen werden, das einen Behälter und ein abnehmbares Element zum Verschließen umfasst, wobei das Element zum Verschließen vorzugsweise ein Element zum Auftragen, d.h. ein Applikator ist.

Ein weiterer Gegenstand der Erfindung ist daher auch eine Vorrichtung zum Auftragen einer Zubereitung, wie oben beschrieben, die einen Behälter, der die Zubereitung aufnehmen kann und ein Halsstück mit Gewinde und Dichtung aufweist, und ein Applikatorelement umfasst, das an seinem einen Ende eine Bürste und an seinem anderen Ende einen Griff, der eine Verschlusskappe bildet, aufweist, wobei der Bürstenkopf einen Durchmesser im Bereich von 5,5 und 6,5 mm und eine Länge im Bereich von 12,5 bis 17,5 mm hat und wobei die Borsten in schraubenförmiger Anordnung radial um ein Mittelstück mit hoher Windungszahl angeordnet sind.

In einer bevorzugten Ausführungsform weist das Auftragssystem einen Behälter auf, der die erfindungsgemäße Zusammensetzung enthält und mit einem Halsstück versehen ist. Das Halsstück weist einen ringförmigen Abstreifer auf. Das Element zum Verschließen des Behälters trägt einen Applikator, der einen Stiel aufweist und an seinem ersten Ende mit einer Bürste als Auftragselement versehen ist und an seinem zweiten Ende mit einem Griff verbunden ist. In geschlossenem Zustand verschließt das Verschlusselement den Behälter, wobei der an dem Verschlusselement angebrachte Applikator in die Zubereitung eintaucht. Zum Auftragen der Zubereitung wird das Verschlusselement herausgezogen, wobei der Applikator über den ringförmigen Abstreifer wandert und dabei überschüssiges Material abstreift. Die Zubereitung kann dann mit der Bürste aufgetragen werden. Da die erfindungsgemäße Zubereitung eine äußerst geringe Viskosität aufweist, ist es möglich, den Behälter in vielfältiger Geometrie auszugestalten, was bei den bekannten Applikatorsystemen für Wimperntusche nicht möglich ist. Übliche Wimperntusche hat eine relativ hohe Viskosität und kann daher nur in zylinderförmigen Behältern aufbewahrt werden, in die der Applikator so eingeführt werden kann, dass er die Masse abnehmen kann. Da die erfindungsgemäße Zubereitung eine solche Viskosität hat, dass die Masse frei fließend ist, kann die Zubereitung auch in bauchigere Gefäße oder Gefäße beliebiger Geometrie eingefüllt werden.

Der Applikator zum Auftragen der Zubereitung erfüllt in einer bevorzugten Ausführungsform die folgenden Bedingungen, damit der Auftrag homogen und beständig wird. Der Applikator ist in der bevorzugten Ausführungsform eine Bürste mit weichen Borsten, damit bei mehrmaligem Auftrag die Borsten die bereits aufgetragene Schicht nicht verletzen und dadurch für Wasser angreifbar machen können. Darüber hinaus ist die Bürste kurz bei großem Durchmesser, damit einzelne Wimpernpartien gezielt angefärbt werden können. Besonders bevorzugt wird eine Bürste mit einem Durchmesser an der Spitze von 5,5 bis 6,5 mm, einer Länge von 12,5 bis 17,5 mm und Nylon als Fasermaterial mit 2,5 mils verwendet. Besonders bevorzugt wird eine Bürste mit großer Windungszahl verwendet.

Erfindungsgemäß wird eine Zubereitung bereitgestellt, die dauerhaft und beständig auf Haut und Haar, Wimpern und/oder Angenbrauen aufgetragen werden kann und dort sehr lange verweilen kann. Sobald die Zubereitung auf die gewünschte Partie aufgetragen ist und getrocknet ist, was nur eine kurze Zeit in Anspruch nimmt, bleibt sie am aufgetragenen Ort haften, ohne auf damit in Berührung kommende Gegenstände überzugehen, ohne in nahe Bereiche auszuwandern oder abzubröseln. Die Zubereitung ist daher ideal geeignet zum Auftragen auf Wimpern, Augenbrauen und Haar. Die Zubereitung kann auf Haar aufgetragen werden, um diesem einen schützenden Überzug und gleichzeitig Fülle und Schwung zu verleihen. In einer bevorzugten Ausführungsform wird die Zubereitung mit Färbemitteln versehen und dient zum Anfärben des keratinischen Materials.

Die Erfindung wird noch durch die folgenden Beispiele erläutert, ohne sie darauf zu beschränken.

### Beispiel 1 - Body-Tattoo

| | |
|---|---|
| Isodecane Acrylates Copolymer | 23,500 |
| Aqua | 19,400 |
| PPG-17 / IPDI / DPMA Copolymer | 20,000 |
| Acrylates Copolymer | 16,500 |
| Propyleneglycol | 3,500 |
| Methyleugenol PEG-8 Dimethicone | 2,200 |
| Adipic Acid / Diethylene Glycol Glycerin Crosspolymer | 2,000 |
| C12 /C15 Alkyl Benzoate | 1,000 |
| Caprylic/Capric Triglyceride | 1,000 |
| Preservatives | 0,750 |
| Dimethicone | 0,150 |
| Pigments | 10,000 |

### Beispiel 2 - Eyeliner

| | |
|---|---|
| Acrylates Copolymer | 30,000 |
| Aqua | 24,450 |
| Polystyrene | 18,000 |
| Caprylic/Capric Triglyceride | 5,000 |
| Propylene Glycol | 2,500 |
| Sorbitol | 1,500 |
| Acetyl Tributyl Citrate | 2,000 |
| Perservatives | 0,750 |
| Sodium Cocoamphoacetate | 0,500 |
| PVP/ Dimethiconyl Polycarbamyl / Polyglcerol Ester | 0,300 |
| Pigments | 15,000 |

### Beispiel 3 - Lidschatten (flüssig)

| | |
|---|---|
| Aqua | 32,750 |
| Ammonium Acrylates Copolymer | 15,000 |
| Polyurethane-1 | 10,000 |
| Adipic Acid / Diethylene Glycol / Glycerin Crosspolymer | 5,000 |
| Dimethicone Copolyol | 4,500 |
| Caprylic/Capric Triglyceride | 4,000 |
| Propylene Glycol | 3,000 |
| Sorbitol | 1,500 |
| Sodium Cocoamphoacetate | 1,000 |
| PEG-180 / Laureth-50 / TMMG Copolymer | 1,000 |
| Preservatives | 0,750 |
| Dimethicone | 0,500 |
| Pigments | 21,000 |

### Vergleichsbeispiel 4 - Eyeliner

| | |
|---|---|
| Polyurethan-7 | 86,250 |
| Dimethicone Copolyol | 4,000 |
| Caprylic/Capric Triglyceride | 3,500 |
| Glycerin | 3,000 |
| Preservatives | 0,750 |
| Dimethicone | 0,500 |
| Pigments | 2,000 |

Eine nach dem Vergleichbeispiel hergestellte - nicht erfindungsgemäße - Zubereitung klebt, hat zu wenig Deckkraft, trägt sich ungleichmäßig auf und versagt im Konservierungsbelastungstest - trotz scheinbar ausreichender Menge an Konservierungsmittel, welche dem Gehalt in den erfindungsgemäßen Beispielen entspricht.

### Beispiel 5 - Wimperntusche

| | Prozent |
|---|---|
| Wasser | q.s. 100 |
| Dicaprylylmaleat | 0,50 |
| Konservierungsmittel | 0,60 |
| Hydrophob modifiziertes Pigment | 10,0 |
| VP/Hexadecen-Copolymer | 0,30 |
| Sorbit | 1,50 |
| Acrylates Copolymer | 20,00 |
| Styrene/Acrylates Copolymer | 12,00 |
| Polyurethan-2 | 28,00 |
| Caprylic/Capric Triglyceride | 1,00 |
| Trimethylsiloxyamodimethicone | 1,00 |
| Entschäumeremulsion | 0,40 |
| 1,4-Butandiol | 3,00 |
| Xanthangum | 0,40 |
| Bentonit | 0,15 |
| Gellan Gum | 0,08 |

### Beispiel 6 - Wimperntusche

| | Prozent |
|---|---|
| Wasser | q.s. 100 |
| Lauret-2-benzoat | 0,70 |
| Konservierungsmittel | 0,70 |
| Hydrophob modifiziertes Pigment | 8,00 |
| Hydrolyzed Weat Protein/Dimethiconecopolyolacetat | 0,40 |
| Sorbit (wässrige Lösung) | 1,50 |
| StyreneNP-Copolymer (wässrige Lösung) | 6,00 |
| Ammonium Acrylates Copolymer (wässrige Lösung) | 25,00 |
| PPG 17/IPDI/DMPA-Copolymer (wässrige Lösung) | 30,00 |
| Caprylic/Capric Triglyceride | 1,00 |
| Glycerin | 1,00 |
| Bis-PEG/PPG-20/20 Dimethicone | 1,00 |
| Entschäumer (wässrige Emulsion) | 0,40 |
| 1,3-Propandiol | 3,50 |
| Hydroxypropylcellulose | 0,11 |
| Xanthan Gum | 0,30 |
| Hectorit | 0,15 |

Aus den obigen Bestandteilen wird eine Wimperntusche hergestellt, in dem die Acrylat-Copolymerdispersion und die Polyurethandispersion vorgelegt und auf 50 bis 55°C vorgewärmt werden und dann die weiteren Inhaltstoffe zugemischt werden. Die Mischung wird so lange gerührt, bis eine homogene Masse vorliegt. Diese Masse kann dann so, wie sie ist, oder abgekühlt abgefüllt werden.

Wenn diese Masse auf Wimpern aufgetragen wird, so entsteht ein glatter gefärbter Überzug, der lange an der Wimper haften bleibt.

Für Zusammensetzungen, die wie oben beschrieben hergestellt wurden, wurde die Viskosität wie folgt bestimmt.

Messgerät: Bohlin CVOR Rheometer,
Schubspannungsrampe 1 bis 1000 Pa aufwärts für Aufnahme einer Fließgrenze
Messsystem: Platte/Platte 20 bis 0,4 mm Spaltabstand
Temperatur: 25°C
Messung ohne Vorscherung
Messzeit: 120 s plus 60 s Vortemperierung auf 25°C
Umrechnung auf Viskositätkurve erfolgt elektronisch

Für erfindungsgemäß hergestellte Rezepturen wurden unter diesen Bedingungen Viskositäten im Bereich unter 2500 mPa.s gemessen.

## Patentansprüche

1. Zubereitung zum Auftragen auf die Haut oder Haar, Wimpern und/oder Augenbrauen auf wässriger Basis enthaltend eine Mischung aus mindestens zwei wässrigen Dispersionen filmbildender Polymere, von denen eines ein Polymer auf Polyurethanbasis und das andere ein Polymer auf Acrylatbasis ist, sowie gegebenenfalls übliche Inhaltsstoffe, wobei die Zubereitung eine Viskosität, gemessen bei 25°C und mit 200 s⁻¹, im Bereich von 0,02 bis 3,8 Pa·s hat, wobei dann, wenn die Zubereitung ein Verdickungsmittel enthält, das Verdickungsmittel nicht pyrogene Kieselsäure ist.

2. Zubereitung nach Anspruch 1, **dadurch gekennzeichnet, dass** als Filmbildner mindestens ein Polymer ausgewählt aus Polyester-Polyurethanen, Polyether-Polyurethanen, Polyurethan-Polyvinylpyrrolidonen, Acrylpolyurethanen und siliconhaltigen Polyurethanen in Form einer wässrigen Dispersion enthalten ist.

3. Zubereitung nach Anspruch 1 oder Anspruch 2, **dadurch gekennzeichnet, dass** die wässrigen Dispersionen 50 bis 90 % der Zubereitung bilden.

4. Zubereitung nach einem der Ansprüche 1 bis 3, **dadurch gekennzeichnet, dass** der Polyurethan-Filmbildner in einem Anteil von 1 bis 60%, jeweils bezogen auf das Gewicht der Zubereitung, enthalten ist.

5. Zubereitung nach einem der vorhergehenden Ansprüche, **dadurch gekennzeichnet, dass** als Acrylates Copolymer ein Acrylpolymer enthalten ist, das durch Polymerisation mindestens eines Monomers, das eine ethylenisch ungesättigte Bindung enthält, ausgewählt aus α,β-ethylenisch ungesättigten Carbonsäuren, α,β-ethylenisch ungesättigten Carbonsäureestern, α,β-ethylenisch ungesättigten Carbonsäureamiden und Mischungen davon gegebenenfalls mit olefinisch ungesättigten Comonomeren erhältlich ist.

6. Zubereitung nach einem der vorhergehenden Ansprüche, **dadurch gekennzeichnet, dass** das Acrylpolymer erhalten wird durch Polymerisation von Monomeren ausgewählt aus der Gruppe bestehend aus Acrylsäure, Methacrylsäure, Crotonsäure, Maleinsäure und Itaconsäure.

7. Zusammensetzung nach einem der vorhergehenden Ansprüche, **dadurch gekennzeichnet, dass** das Acrylpolymer ausgewählt ist aus Aryl- und Alkylacrylaten und Mischungen davon.

8. Zubereitung nach einem der vorhergehenden Ansprüche, **dadurch gekennzeichnet, dass** als Acrylpolymer eine Mischung aus Acrylates Copolymer und Styrol/Acrylcopolymeren enthalten ist.

9. Zubereitung nach einem der vorhergehenden Ansprüche, **dadurch gekennzeichnet dass**, in den Polymerdispersionen die Polymere als Teilchen mit einer Größe von 10 bis 1000 nm vorhanden sind.

10. Zubereitung nach einem der vorhergehenden Ansprüche, **dadurch gekennzeichnet, dass** die in den Polymerdispersionen enthaltenen Polymere Filmbildungstemperaturen unter 30°C haben.

11. Zubereitung nach einem der vorhergehenden Ansprüche, **dadurch gekennzeichnet, dass** mindestens 30 % der in den Polymerdispersionen enthaltenen im getrockneten Zustand wasserfesten Polymere oder Copolymere eine Glasübergangstemperatur unter 10°C haben.

12. Zubereitung nach einem der vorhergehenden Ansprüche, **dadurch gekennzeichnet, dass** 5 bis 30 % einer oder mehrerer der Dispersionen von statistischen oder Blockcopolymeren einen Block oder Abschnitt aufweisen, dessen Glasübergangstemperatur im getrockneten Zustand höher als 30°C ist.

13. Zubereitung nach einem der vorhergehenden Ansprüche, **dadurch gekennzeichnet, dass** als übliche Inhaltsstoffe Weichmacher, Netzmittel, Entschäumer, Färbemittel, Konservierungsmittel, Duft- und/oder Aromastoffe enthalten sind.

14. Zubereitung nach einem der Ansprüche 1 bis 13, **dadurch gekennzeichnet, dass** ein oder mehrere Pigmente enthalten sind.

15. Zubereitung nach Anspruch 14, **dadurch gekennzeichnet, dass** zusätzlich mindestens ein Verdicker enthalten ist.

16. Zubereitung nach Anspruch 15 **dadurch gekennzeichnet, dass** zur Verdickung natürliche und/oder synthetische und/oder modifizierte Polysaccharide, mineralische und/oder modifizierte mineralische Verdicker, Silicatverdicker, Cellulosen und modifizierte Cellulosen, nichtionische Assoziativverdicker und/oder Copolymerverdicker enthalten sind.

17. Zubereitung nach Anspruch 15 oder Anspruch 16, **dadurch gekennzeichnet, dass** mindestens ein Polysaccharid, mindestens ein mineralischer Verdicker und mindestens eine pflanzliche Gumme enthalten ist.

18. Zubereitung nach einem der Ansprüche 14 bis 17, **dadurch gekennzeichnet, dass** zur Verdickung eine Mischung aus Cellulose oder einem Cellulosederivat, ein silicatischer Verdicker und Xanthamgummi enthalten ist.

19. Zubereitung nach Anspruch 18, **dadurch gekennzeichnet, dass** Cellulose, Silicat und Xanthamgummi in einem Verhältnis von 1:1:4 bis 1:2:6 enthalten sind.

20. Zubereitung nach einem der Ansprüche14 bis 19, **dadurch gekennzeichnet, dass** der Verdicker in einem Anteil von 0,2 bis 20%, insbesondere 0,2 bis 5%, jeweils bezogen auf das Gewicht der Zusammensetzung enthalten ist.

21. Verwendung einer Zubereitung nach einem der Ansprüche 1 bis 20 zum Auftragen auf Haut oder Haar, Wimpern und/oder Augenbrauen.

22. Verwendung nach Anspruch 21 zum Schminken der Augen und Augenwimpern.

23. Vorrichtung zum Auftragen einer Zubereitung zum Auftragen auf die Haut, Haar, Wimpern und/oder Augenbrauen auf wässriger Basis enthaltend eine Mischung aus mindestens zwei wässrigen Dispersionen filmbildender Polymere, von denen eines ein Polymer auf Polyurethanbasis und das andere ein Polymer auf Acrylatbasis ist, sowie gegebenenfalls übliche Inhaltsstoffe, wobei die Zubereitung eine Viskosität, gemessen bei 25°C und mit 200 s⁻¹, im Bereich von 0,02 bis 3,8 Pa·s hat, wobei dann, wenn die Zubereitung ein Verdickungsmittel enthält, das Verdickungsmittel nicht pyrogene Kieselsäure ist, umfassend einen Behälter, der die Zubereitung aufnehmen kann und ein Halsstück mit Gewinde und Dichtung aufweist, und ein Applikatorelement, das an seinem einen Ende eine Bürste und an seinem anderen Ende einen Griff, der eine Verschlusskappe bildet aufweist, wobei der Bürstenkopf einen Durchmesser im Bereich von 5,5 und 6,5 mm und eine Länge im Bereich von 12,5 bis 17,5 mm hat und wobei die Borsten in schraubenförmiger Anordnung radial um ein Mittelstück mit hoher Windungszahl angeordnet sind.

## Claims

1. A preparation for application to the skin, hair, eyelashes and/or eyebrows, which is water-based and contains a mixture of at least two aqueous dispersions of film-forming polymers, one of which is a polyurethane-based polymer and the other an acrylic-based polymer, and optionally conventional ingredients, the preparation having a viscosity, measured at 25°C and 200 s⁻¹, in the range of 0.02 to 3.8 Pa·s, wherein, when the preparation contains a thickener, the thickener is not pyrogenic silica.

2. The preparation according to claim 1, **characterised in that** at least one polymer selected from polyester polyurethanes, polyether polyurethanes, polyurethane polyvinyl pyrrolidones, acrylic polyurethanes and silicone polyurethanes or mixtures thereof is contained in the form of an aqueous dispersion as the polyurethane film former.

3. The preparation according to claim 1 or claim 2, **characterised in that** the aqueous dispersions form 50 to 90% of the preparation.

4. The preparation according to one of claims 1 to 3, **characterised in that** the polyurethane film former is contained in a proportion of 1 to 60%, based on the weight of the preparation in each case.

5. The preparation according to one of the above claims, **characterised in that** an acrylic polymer that is obtainable by polymerisation of at least one monomer containing an ethylenically unsaturated bond, selected from α,β-ethylenically unsaturated carboxylic acids, α,β-ethylenically unsaturated carboxylic acid esters, α,β-ethylenically unsaturated carboxylic acid amides and mixtures thereof, optionally with olefinically unsaturated comonomers, is contained as the acrylic copolymer.

6. The preparation according to one of the above claims, **characterised in that** the acrylic polymer is obtained by polymerisation of monomers selected from the group consisting of acrylic acid, methacrylic acid, crotonic acid, maleic acid and itaconic acid.

7. A composition according to one of the above claims, **characterised in that** the acrylic polymer is selected from aryl and alkyl acrylates and mixtures thereof.

8. The preparation according to one of the above claims, **characterised in that** a mixture of acrylates copolymer and styrene/acrylic copolymers is contained as the acrylic polymer.

9. The preparation according to one of the above claims, **characterised in that** the polymers are present in the polymer dispersions as particles with a size of 10 to 1000 nm.

10. The preparation according to one of the above claims, **characterised in that** the polymers contained in the polymer dispersions have film-forming temperatures of less than 30°C.

11. The preparation according to one of the above claims, **characterised in that** at least 30% of the polymers or copolymers contained in the polymer dispersions, which are water-resistant in the dried state, have a glass transition temperature of less than 10°C.

12. The preparation according to one of the above claims, **characterised in that** 5 to 30% of one or more of the dispersions of random or block copolymers have a block or section the glass transition temperature of which, in the dried state, is greater than 30°C.

13. The preparation according to one of the above claims, **characterised in that** plasticisers, wetting agents, defoamers, colorants, preservatives, fragrances and/or flavourings are contained as conventional ingredients.

14. The preparation according to one of claims 1 to 13, **characterised in that** one or more pigments are contained.

15. The preparation according to claim 14, **characterised in that**, in addition, at least one thickener is contained.

16. The preparation according to claim 15, **characterised in that** natural and/or synthetic and/or modified polysaccharides, mineral and/or modified mineral thickeners, silicate thickeners, celluloses and modified celluloses, non-ionic associative thickeners and/or copolymer thickeners are contained for thickening purposes.

17. The preparation according to claim 15 or claim 16, **characterised in that** at least one polysaccharide, at least one mineral thickener and at least one vegetable gum is contained.

18. The preparation according to one of claims 15 to 17, **characterised in that** a mixture of cellulose or a cellulose derivative, a silicate thickener and xanthan gum is contained for thickening purposes.

19. The preparation according to claim 18, **characterised in that** cellulose, silicate and xanthan gum are contained in a ratio of 1:1:4 to 1:2:6.

20. The preparation according to one of claims 14 to 19, **characterised in that** the thickener is contained in a proportion of 0.2 to 20%, particularly 0.2 to 5%, based in each case on the weight of the composition.

21. The use of a preparation according to one of claims 1 to 20 for application on to skin, hair, eyelashes and/or eyebrows, particularly on to keratinous material.

22. The use according to claim 21 for making up the eyes and eyelashes.

23. A device for the application of a preparation for application on to the skin, hair, eyelashes and/or eyebrows which is water-based and contains a mixture of at least two aqueous dispersions of film-forming polymers, one of which is a polyurethane-based polymer and the other an acrylic-based polymer, and optionally conventional ingredients, the preparation having a viscosity, measured at 25°C and 200 s⁻¹, in the range of 0.02 to 3.8 Pa·s, wherein, when the preparation contains a thickener, the thickener is not pyrogenic silica, comprising a container which can hold the preparation and a neck piece with a thread and seal, and an applicator element having, at one end thereof, a brush and, at the other end thereof, a handle which forms a closure cap, the brush head having a diameter in the range of 5.5 to 6.5 mm and a length in the range of 12.5 to 17.5 mm, and the bristles being arranged in a spiral arrangement radially around a central piece with a high number of turns per unit length.

## Revendications

1. Préparation destinée à une application sur la peau, les cheveux, les cils et/ou les sourcils, à base aqueuse, contenant un mélange d'au moins deux dispersions aqueuses de polymères filmogènes, parmi lesquels un est un polymère à base de polyuréthane et l'autre un polymère à base d'acryle, ainsi qu'éventuellement des ingrédients usuels, dans laquelle la préparation a une viscosité, mesurée à 25 °C et avec 200 s⁻¹, dans la gamme de 0,02 à 3,8 Pa.s et dans laquelle ensuite, lorsque la préparation contient un épaississant, l'épaississant est de l'acide silicique non pyrogène.

2. Préparation selon la revendication 1, **caractérisée en ce qu'**elle contient en tant qu'agent filmogène de polyuréthane au moins un polymère sélectionné parmi des polyester-polyuréthanes, des polyéther-polyuréthanes, des polyuréthane-polyvinylpyrrolidones, des acrylpolyuréthanes et des polyuréthanes siliconés ou des mélanges de ceux-ci, sous la forme d'une dispersion aqueuse.

3. Préparation selon la revendication 1 ou la revendication 2, **caractérisée en ce que** les dispersions aqueuses forment 50 à 90 % de la préparation.

4. Préparation selon l'une des revendications 1 à 3, **caractérisée en ce que** l'agent filmogène de polyuréthane est contenu dans une proportion comprise entre 1 et 60 %, respectivement sur la base de la masse de la préparation.

5. Préparation selon l'une des revendications précédentes, **caractérisée en ce qu'**elle contient en tant que copolymère d'acryle un polymère d'acryle qui peut être obtenu par polymérisation d'au moins un monomère contenant une liaison insaturée en éthylène, sélectionné parmi des acides carboxyliques insaturés en α,β-éthylène, des esters d'acide carboxylique insaturés en α,β-éthylène, des amides d'acide carboxylique insaturés en α,β-éthylène et des mélanges de ceux-ci, éventuellement avec des co-monomères insaturés en oléfine.

6. Préparation selon l'une des revendications précédentes, **caractérisée en ce que** le polymère d'acryle est obtenu par polymérisation de monomères sélectionnés dans le groupe composé de l'acide acrylique, de l'acide méthacrylique, de l'acide crotonique, de l'acide maléique et de l'acide itaconique.

7. Composition selon l'une des revendications précédentes, **caractérisée en ce que** le polymère d'acryle est sélectionné parmi des acrylates d'aryle et d'alkyle, et des mélanges de ceux-ci.

8. Préparation selon l'une des revendications précédentes, **caractérisée en ce qu'**elle contient en tant que polymère d'acryle un mélange d'un copolymère d'acrylate et de copolymères de styrène/acryle.

9. Préparation selon l'une des revendications précédentes, **caractérisée en ce que** dans les dispersions polymères, les polymères sont présents sous la forme de particules ayant une taille comprise entre 10 et 1 000 nm.

10. Préparation selon l'une des revendications précédentes, **caractérisée en ce que** les polymères contenus dans les dispersions de polymères ont des températures de formation de film inférieures à 30 °C.

11. Préparation selon l'une des revendications précédentes, **caractérisée en ce qu'**au moins 30 % des polymères ou copolymères contenus dans les dispersions polymères et résistant à l'eau à l'état sec ont une température de transition vitreuse inférieure à 10 °C.

12. Préparation selon l'une des revendications précédentes, **caractérisée en ce que** 5 à 30 % d'une ou de plusieurs des dispersions de copolymères statistiques ou séquencés présentent un bloc ou segment dont la température de transition vitreuse à l'état sec est supérieure à 30 °C.

13. Préparation selon l'une des revendications précédentes, **caractérisée en ce qu'**elle contient en tant qu'ingrédients usuels des plastifiants, des agents mouillants, des agents anti-mousse, des colorants, des conservateurs, des parfums et/ou des arômes.

14. Préparation selon l'une des revendications 1 à 13, **caractérisée en ce qu'**elle contient un ou plusieurs pigments.

15. Préparation selon la revendication 14, **caractérisée en ce qu'**elle contient en outre au moins un épaississant.

16. Préparation selon la revendication 15, **caractérisée en ce qu'**elle contient pour l'épaississement des polysaccharides naturels et/ou synthétiques et/ou modifiés, des épaississants minéraux et/ou minéraux modifiés, des épaississants au silicate, des celluloses et des celluloses modifiées, des épaississants d'association non ioniques et/ou des épaississants de type copolymères.

17. Préparation selon la revendication 15 ou la revendication 16, **caractérisée en ce qu'**elle contient au moins un polysaccharide, au moins un épaississant minéral et au moins une gomme végétale.

18. Préparation selon l'une des revendications 15 à 17, **caractérisée en ce que** pour l'épaississement, elle contient un mélange de cellulose ou d'un dérivé de cellulose, d'un épaississant au silicate et de gomme de xanthane.

19. Préparation selon la revendication 18, **caractérisée en ce qu'**elle contient la cellulose, le silicate et la gomme de xanthane dans un rapport compris entre 1:1:4 et 1:2:6.

20. Préparation selon l'une des revendications 14 à 19, **caractérisée en ce qu'**elle contient l'épaississant dans une proportion comprise entre 0,2 et 20 %, en particulier entre 0,2 et 5 %, respectivement sur la base de la masse de la composition.

21. Utilisation d'une préparation selon l'une des revendications 1 à 20 pour une application sur la peau, les cheveux, les cils et/ou les sourcils, en particulier sur un matériau kératineux.

22. Utilisation selon la revendication 21 destinée au maquillage des yeux et des cils.

23. Dispositif d'application d'une préparation, destinée à une application sur la peau, les cheveux, les cils et/ou les sourcils, à base aqueuse, contenant un mélange d'au moins deux dispersions aqueuses de polymères filmogènes, parmi lesquels un polymère est à base de polyuréthane et l'autre polymère est un polymère à base d'acryle, ainsi qu'éventuellement des ingrédients usuels, la préparation présentant une viscosité, mesurée à 25 °C et à 200 s⁻¹, dans la gamme comprise entre 0,02 et 3,8 Pa.s, suite à quoi, lorsque la préparation contient un épaississant, l'épaississant est un acide silicique non pyrogène, comportant un conteneur qui peut loger la préparation et présente un col avec filetage et garniture d'étanchéité, et un élément applicateur qui présente à l'une de ses extrémités une brosse et à l'autre de ses extrémités une poignée, qui forme un couvercle, la tête de la brosse ayant un diamètre dans la gamme comprise entre 5,5 et 6,5 mm et une longueur dans la gamme comprise entre 12,5 et 17,5 mm, et les soies étant disposées selon une disposition en forme d'hélice radialement autour d'une pièce centrale et ayant un grand nombre de spires.
